(19) Europäisches Patentamt / European Patent Office / Office européen des brevets

(11) **EP 3 984 443 A1**

(12) **EUROPEAN PATENT APPLICATION**

(43) Date of publication:
**20.04.2022 Bulletin 2022/16**

(21) Application number: **20201621.8**

(22) Date of filing: **13.10.2020**

(51) International Patent Classification (IPC):
*A61B 5/00* (2006.01)     *G16H 20/17* (2018.01)
*G16H 40/63* (2018.01)

(52) Cooperative Patent Classification (CPC):
**A61B 5/14532; A61B 5/4839; A61B 5/7264;
G16H 20/17; G16H 40/63;** A61M 5/14248;
A61M 5/1723

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Diabeloop**
**38028 Grenoble Cedex 1 (FR)**

(72) Inventor: **HUNEKER, Erik**
**92800 PUTEAUX (FR)**

(74) Representative: **Fidal Innovation**
**4-6 avenue d'Alsace**
**92400 Courbevoie (FR)**

(54) **COMPUTERIZED SYSTEM FOR REPEATEDLY DETERMINING A VALUE OF A CONTROL PARAMETER OF A FLUID INFUSION SYSTEM**

(57)    The computerized system repeatedly determines a value of a control parameter of a fluid infusion system for a diabetic patient, based on a data-based processing user-parametered with a target parameter and at least one aggressiveness parameter.

[Fig. 3]

**Description**

FIELD OF THE INVENTION

The present invention relates to computerized systems for repeatedly determining a value of a control parameter of a fluid infusion system for a diabetic patient.

TECHNOLOGICAL BACKGROUND

More precisely, the invention relates to the control of infusion of fluid into diabetic patients.

In the field of diabetes, it is known to evaluate the concentration of blood glucose, and to inject a quantity of insulin as a function of the measured concentration. Recently, so-called "closed-loop" systems were developed, where a processor is programmed to repeatedly evaluate a volume rate of insulin to be injected, based on patient data, and to control the injection of insulin based on this evaluation. In addition, the processor can be programmed to evaluate a volume of insulin to be injected in some special circumstances, in particular meals. The volume can be injected to the patient, subject to the patient's approval. Such systems are also called "semi closed-loop", because of the necessary declaration by the patient of some of these special circumstances.

Determination of a correct quantity or rate of insulin to be injected is very complex, for many different reasons. One reason is the very different biological nature of all patients. Another reason is the very large number of factors which affect the influence of the injected insulin to the blood glucose concentration. There are many other reasons.

Hence, the quantity or rate of insulin determined by the processor is necessarily a trade-off between various competing requirements. The processor will apply a more-or-less complex model to take into account this large number of factors.

Most often, the model can be parametered for the patient. This parametrization can be performed when the patient first uses the system, and can be performed using medical knowledge by a doctor specialized in diabetes. The patient-specific parametered model will be used by the controller to regulate the injection of insulin in a patient-specific way.

The patient themselves may also influence the result of the model. For example, the patient himself may define a glycemic target at a given value, which is the targeted value of blood glucose concentration at a given future time. The processor will determine the quantity or rate of insulin to be injected at the present time in order for the blood glucose concentration at the future time to be close to the defined target. Another option is to define a target range, rather than a target (so-called "control-to-range" algorithms). According to this definition, the processor will determine the quantity or rate of insulin to be injected at the present time in order for the blood glucose concentration at a given future time to be inside the defined target range.

Another parameter which influences the system is the aggressiveness. Aggressiveness might be understood as the intensity of the instantaneous action of the system to regulate the phenomenon. A system with low aggressiveness will have, at a given time, little effect on the phenomenon, and it will take time to reach the target, which might eventually even not be reached if aggressiveness is too low. To the contrary, a system with high aggressiveness will more certainly reach the target, but may overcome the target, which may raise complications.

WO 2017/209902 is a document which describes an artificial pancreas with a "control-to-range" regulation. Aggressiveness is mentioned in this document, which discloses various methods where aggressiveness can be used to regulate the infusion of insulin of the patient.

According to one embodiment of this document, as described in its paragraph 44, aggressiveness is taken into account through a parameter $\alpha_{hyper}$ which is used in defining a cost function related to the risk of hyperglycemia: $h(g) = h(g)_{hyper}$ in the complex below scenario :

$$h(g)_{hyper} = \max(\alpha_{hyper} \cdot \alpha \, (\log(\max(g - \Delta g_{hyper} - \max(\Delta g_{hypo}, 0), 1))^c - \beta), 0)$$

$$h(g)_{hypo} = \min(\alpha \, (\log(\max(g - \Delta g_{hypo}, 1))^c - \beta), 0)$$

where

$$h(g) = h_{MAX} \text{ if } g - \Delta g_{hyper} - \max(\Delta g_{hypo}, 0) \geq g_{MAX};$$

$$h(g) = h_{MIN} \text{ if } g - \Delta g_{hypo} \leq g_{MIN};$$

$$h(g) = h(g)_{hyper} \text{ if } h(g)_{hypo} \geq 0;$$

$$h(g) = h(g)_{hypo} \text{ if } h(g)_{hypo} < 0$$

where g is the blood glucose value (mg/dl) shown on the x-axis, h(g) is the corresponding hazard value shown on the y-axis, $\Delta g_{hyper}$ is a hyperglycemic shift, $\Delta g_{hypo}$ is a hypoglycemic shift, $h_{MAX}$ is a maximum hazard, $h_{MIN}$ is a minimum hazard, $\alpha_{hyper}$ is the hyperglycemic control aggressiveness, and $\alpha$, $\beta$ and c are process variables.

According to a second embodiment of this document, as described in its paragraph 50, aggressiveness of the control-to-range algorithm would be adjusted by modifying the allowed glucose acceleration. However, glucose acceleration would result from many parameters and from the metabolism of the patient, and it is in fact not possible to control the allowed glucose acceleration.

According to a third embodiment of this document, as described in its paragraph 65, aggressiveness of the control-to-range algorithm is related to the uncertainty in the measurement of blood glucose concentration.

However, a correct parametrization of the aggressiveness seems very difficult in these embodiments, since aggressiveness is either integrated as one factor in complex multi-factors equations, or is indirectly defined through another parameter.

Another document which mentions aggressiveness of an artificial pancreas is WO 2017/205,380. According to this document, the patient is able to enter a "fear of hypoglycemia index (FHI)". This index seems to be used in order to modify the target of the algorithm.

The invention thus aims at a simple, safe and efficient control of an artificial pancreas with an aggressiveness factor.

SUMMARY OF THE INVENTION

Thus, the invention relates to a computerized system for repeatedly determining a value of a control parameter of a fluid infusion system for a diabetic patient, wherein the computerized system comprises a processor for repeatedly determining said value based on a data-based processing user-parametered with a target parameter and at least one aggressiveness parameter.

The invention allows the user to simply parameter the system. Further, the user-defined parameters are easy to understand for the user, which can easily adapt the configuration of the system according to its current needs. In particular, the patient has the possibility to express their preference in terms of treatment aggressiveness, and this will directly impact the aggressiveness of the treatment.

According to various aspects, one or more of the following features may be implemented.

According to some embodiments, the aggressiveness parameter relates to the acceleration of the action of the control parameter to force the value of the analyte toward the value of the target parameter.

According to some embodiments, the processor determines a basal flow value by applying said data-based processing on detected analyte data.

According to some embodiments, the processor determines a bolus quantity value.

According to some embodiments, the processor determines a bolus value user-triggered to compensate for a meal.

According to some embodiments, the processor determines a schedule for delivery of the bolus value.

According to some embodiments, the processing comprises one or more of a model-predictive control module, an expert module, an activity-handling module, notably a meal-handling module.

According to some embodiments, the processing comprises two or more of a model-predictive control module, an expert module, an activity-handling module, notably one or more meal-handling modules, wherein the aggressiveness parameter of two different modules are different, and notably wherein the aggressiveness parameter of two different modules determining a basal value are different and/or the aggressiveness factors of two different modules determining a bolus value are different.

According to another aspect, the invention relates to a fluid infusion system for a diabetic patient comprising:

- a sensor system for acquiring patient data and storing said patient data in a database,
- a computerized system according to any of claims 1 to 8, and,
- a pump controlling infusion of said fluid into the patient based on said control parameter.

According to some embodiments, the fluid infusion system further comprises a user interface adapted to enable parametring said target parameter and said at least one aggressiveness parameter.

According to another aspect, the invention relates to a computer program adapted to repeatedly determine a value of a control parameter of a fluid infusion system for a diabetic patient, by making a processor repeatedly determining said value based on a data-based processing user-parametered with a target parameter and at least one aggressiveness parameter.

BRIEF DESCRIPTION OF THE DRAWINGS

Embodiments of the invention will be described below, in relation to the following drawings :

Fig. 1    shows a medical system.

Fig. 2    shows a decision module.

Fig. 3    shows an interface window.

On the drawings, the same reference signs show the same or similar objects.

In the present detailed description, and according to the WHO Expert Committee on Biological Standardization, one international unit of insulin (1 U) is defined as the "biological equivalent" of 34.7 micrograms ($\mu$g) pure crystalline insulin. This unit is the relevant unit for discussing a quantity of insulin to be infused to a patient, and can not be converted to the International System of Units, because the conversion would depend on which insulin is being used. For the sake of the disclosure of the present invention, it is important that quantities of insulin be expressed in a system meaningful for the invention, the readers and the scientific community.

DETAILED DESCRIPTION

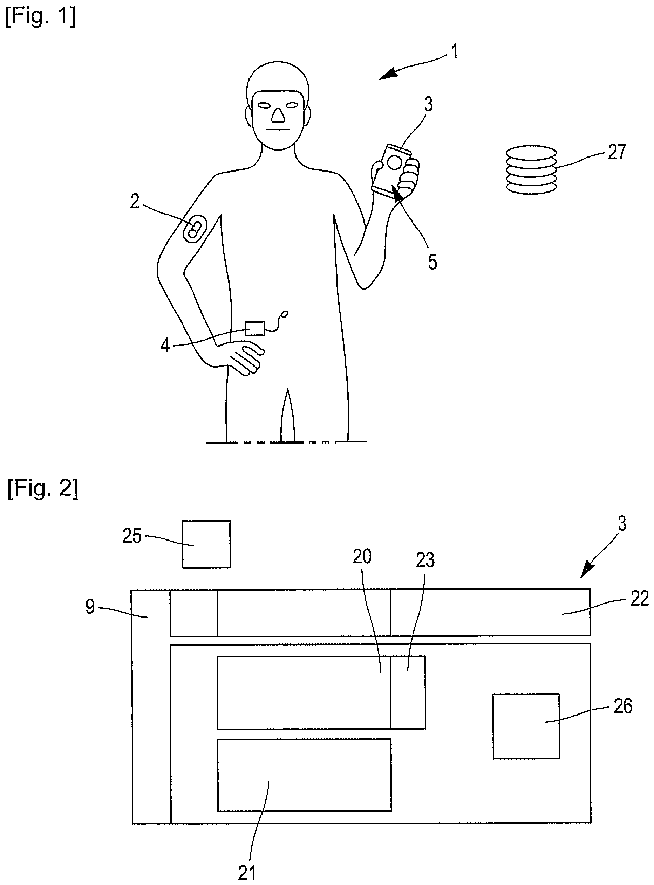

Figure 1 typically shows a medical system 1. The medical system 1 comprises a data acquisition system 2, a data processing system 3, and an active system 4.

The medical system 1 comprises a communication system adapted to rule communications between the various components of the system. In particular, in the present example, the communication system enables communication between the data acquisition system 2 and the data processing system 3, by which data acquired by the data acquisition system 2 can be transferred to the data processing system 3 for processing. The communication system enables communication between the data processing system 3 and the active system 4, by which control parameters of the active system 4 determined by the data processing system 3 can be transferred to the active system 4 to control the active system 4. The communication system is for example a wireless communication system operating according to one or more communication standards, such as Bluetooth.

The data acquisition system 2 acquires analyte patient data. The data acquisition system 2 may for example comprise a continuous glucose monitoring system, adapted to regularly estimate a concentration of blood glucose in the patient's blood. Various technologies are available. Importantly, this estimation is performed repeatedly, for example in the order of time of the minute. The acquired analyte data may be pre-processed, and is stored in a database 27 accessible by the data processing system 3.

The data processing system 3 is part of a hand-held device 5. The data processing system 3 comprises a processor which runs one or more computer programs in order to determine a control parameter for the active system 4.

The data-processing system 3 is adapted to determine a value of a control parameter of the active system 4. As described here, "a value of a control parameter" may be any kind of value of one or more control parameters, such as Boolean, integer, real values, look-up tables, function(s) of time or function(s) of another parameter, ... The control parameter may be a single control parameter, or a combined control parameter, for example a pair of independent control parameters.

The data-processing system 3 is adapted to repeatedly determine the value for the control parameter. For example, it comprises a clock scheduling repeated determination steps. Alternatively, the determination of the value for the control parameter is triggered by the reception of measurement data from the data acquisition system 2, or by other triggers, such as an activity declaration.

The determination may be based on analyte data acquired by the data acquisition system 2, and on one or more computerized modules determining a value for the control parameters based on this data. The determination may alternatively or in addition be based on past determined values for the control parameter.

In the present example, the data-processing system operates four different modules arranged according to a pre-established decision module, as shown on Figure 2. However, the present invention may be applied to any other kind of data-processing system which determines a control value for the active system 4 based on a data acquisition system 2 and one or more computerized modules.

According to one example, the data-processing module comprises a data-based control module. The data-based control module is designed to determine a value for the control parameter based on a data-driven model. The data-driven model is based on data representative of the relationship between the data measured by acquisition systems or declared by the patient and control parameter in other situations. According to one example, the control module 20 is a data-based module set up as a model predictive control module. The model predictive control module comprises a biological model of the patient designed to model the interaction of infused insulin and blood glucose concentration in the patient. This model is patient-specific, and depends on recent observation data of the patient (in terms of measured data of blood glucose concentration and associated insulin infusion control value) and may have been originally parametrized. Notably, the physiological parameters of the patient in the model may comprise glucose transfer rates between various physiological compartments, time to peak glucose absorption ingested by the patient, etc... The model predictive control module takes into account a history of blood glucose concentration of the patient and of infused insulin and other factors (declared meals, declared physical exercises, etc ...) to predict a likely future value for the blood glucose concentration of the patient depending on a value of the control parameter to be set. The control parameter is for example a rate of insulin to be injected. The value of the control parameter will be determined based on a pre-defined optimum. If, for example, the pre-defined optimum is to have a target value of 100 mg/dl for the predicted future value of the blood glucose concentration three hours after the calculation time, it will determine the value of the control parameter which will provide a predicted future value of the blood glucose concentration three hours after the calculation time which will be closest to this target. "Closest" might be evaluated under a number of various definitions of a distance.

According to one example, the data-processing module comprises a so-called expert module 21, which is rule-based. For example, the expert module would apply pre-determined rules on available data. The available data taken into account by the expert module may comprise or be limited to the current and/or past level of blood glucose concentration, and the history of the control command. One example of such rules may comprise a look-up table defining a value of the control parameter depending on various situations. For example, the look-up table may comprise entries as to intervals for the current level of blood glucose concentration and/or the current trend of blood glucose concentration , and/or a short-term forecast of the blood glucose concentration, and selects a suitable equation to be applied, which will provide a value of the control parameter for each of the pre-defined interval entries.

According to one example, the data-processing module comprises a safety module 22, which will apply some specific pre-determined rules in case of determined safety risks. For example, the determined safety risk may be a risk of hypoglycemia, or a risk of hyperglycemia. The safety risk might be determined based on the available data, such as, for example, current level and/or trend of blood glucose concentration ., short-term forecast of the blood glucose concentration and/or long-term forecast of the blood glucose concentration.

According to one example, the data-processing module comprises an activity-handling module 26. Examples of activities handled by the activity-handling module include meals. The activity-handling module will apply some specific pre-determined rules depending on the activity.

According to one example, the data-processing module comprises one-or more auto-learning based modules. The auto-learning based module might be updated from time to time. This means that, at a given time, this module determining the value for the control parameter is not the originally-parametered module , but a module based on this originally-parametered module, which was updated in the meanwhile. The updating of the module may be based on historical data of the patient, and/or of other patients.

According to one example, the auto-learning module 25, which updates the auto-learning based module, is run less frequently than the determination of the value for the control parameter.

In particular, for example, the model predictive control module 20 might be an auto-learning based module.

At any given time, some of the above-described modules may be operated under a decision module 9.

For example, the safety module 22 is operated first. If the decision module 9 estimates that a safety risk is high, it will apply the safety module 22 to determine the value of the control parameter. If the decision module 9 estimates that the safety risk is low, it will move to the other modules. The values for "High" and "low" might be predetermined and/or parametrable, or even auto-learned as discussed above.

The value of the control parameter might be determined as a function of the value of the control parameters determined by the expert module 21 and the model predictive control module 20.

For example, the decision module 9 may comprise a confidence assessment module 23 which assesses a confidence in the value for the control parameter determined by the auto-learned module. For example, the confidence assessment module 23 applies the auto-learned module to past data, compares the result of applying the auto-learned module to past data with the actual measured data, and assesses the level of confidence in the auto-learned module based on this comparison. In such case, the value of the control parameter might be determined as a function of the value of the control parameters determined by the expert module 21 and the model predictive control module 20, and as a function of the confidence assessment module 23.

For example, the function rendered by the confidence assessment module 23 might be that, if the confidence is high, the auto-learned module is applied to determine the value for the control parameter and, if the confidence is low, the non-auto-learned module is applied to determine the value for the control parameter. "High" and "low" confidences might be based on pre-determined and/or parametrable thresholds. Alternatively, other functions might be used.

The above scheme is programmed to operate as default, and determines a value for a control parameter of the active system 4.

The system, and in particular the hand-held device 5, provides an interface enabling the patient to configure some parameters of the system. The patient is sometimes also called the user, and s/he has little medical training, and has access only to a few parameters of the system. The doctor may have access to other parameters of the system, but the user may not have access to the same parametring interface as the doctor. This might be provided by providing different interfaces accessible by keywords or other authentication technologies.

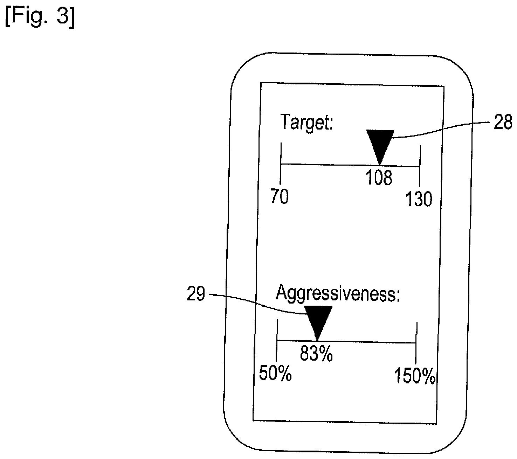

Fig. 3 shows an interface window accessible by the patient for the parametring of target and aggressiveness. For example, in the upper part of the window, there is a scale for the target between a min value and a max value, which may be fixed, for example respectively at 70 and 130 mg/dl, and the patient may move an arrow 28 along the scale between the min and max value to the desired target value, which is displayed. According to the shown example, the displayed selected target value is 108. For example, in the lower part of the window, there is a scale for the aggressiveness between a min value and a max value, which may be fixed, for example respectively at 50% and 150% (relative scale to an average aggressiveness of 100%), and the patient may move an arrow 29 along the scale between the min and max value to the desired aggressiveness value, which is displayed. According to the shown example, the displayed selected aggressiveness value is 83%.

Even though the above description shows a single aggressiveness scale, such a scale could be provided for each of the user-parametrable aggressivenesses as defined below.

According to one embodiment, target (or target range) and aggressiveness(es) are the only parameters that the patient may define through its user interface.

According to one embodiment, one of the modules operates in basal infusion mode. "Basal" is used to define a continuous mode of infusion of insulin where the control parameter of the insulin flow is determined as a value of flow (or rate) and, in the present example, is lower than 5 U/h.

Control of the basal rate by the Model-predicitive controller

One example of the modules operating in basal infusion mode is the model predictive control module 20. Under this module, the aggressiveness is taken into account according to the following formula:

$$Basal = min\ (Basal_{MAX};\ Basal_{MPC}),$$

where:

$Basal_{MPC}$ is the value of the control parameter defined taking into account the physiological model of the patient, which does not take into account the aggressiveness,

$$Basal_{MAX} = \alpha_m\ .\ f\ (BasalRef_M,\ IS,\ \underline{gl},\ Target)$$

where:

$Basal_{MAX}$ is the maximal value for the control parameter,

$\alpha_m$ is the aggressiveness factor,

$BasalRef_M$ is a pre-defined reference value for the infusion of basal insulin for the patient for this module,

IS is the sensitivity to insulin of the patient (for example, this parameter may be predefined),

Target is the above-defined target blood glucose concentration,

$\underline{gl}$ is the short-term predicted blood glucose concentration - Short-term corresponds for example to any predefined future time between 5 minutes and 1 hour from the instant time. and

f is a multiplicative factor determined as a function of the sensitivity to insulin of the patient IS, the target blood glucose concentration "Target", and the predicted blood glucose concentration $gl$ at short term. The function f is a rising function of the predicted blood glucose concentration, for example continuous or stepwise. According to this function, $Basal_{MAX}$ will be raised if predicted short-time blood glucose concentration is high. This allows for more insulin to be injected if there is a prediction that blood glucose concentration will be higher in the short-term. This function thus enables to infuse less insulin if the patient has a current low blood glucose concentration, which reduces the risk of hypoglycemia for the patient. In addition, this function takes into account the lower sensibility of the patient to insulin by high blood glucose concentration.

Control of the basal rate bv the expert module controller

According to one example, the expert module 21 operates in basal infusion mode. Under this module, the aggressiveness is taken into account according to the following formula:

$$Basal = min\ (Basal_{MAX};\ Basal_{EM}),$$

where:
$Basal_{EM}$ is the value of the control parameter defined using the expert module, which does not take into account the aggressiveness,

$$Basal_{MAX} = \alpha_e\ .\ g\ (BasalRef_E,\ IS,\ gl,\ Target)$$

where:

$Basal_{MAX}$ is the maximal value of the control parameter,

$\alpha_e$ is the aggressiveness factor,

$BasalRef_E$ is a pre-defined reference value for the infusion of basal insulin for the patient for this model,

IS is the sensitivity to insulin of the patient (for example, this parameter may be predefined),

Target is the above-defined target blood glucose concentration,

$gl$ is the short-term predicted blood glucose concentration - Short-term corresponds for example to any future time between 5 minutes and 1 hour from the instant time. and

g is a multiplicative factor defined as a function of $BasalRef_E$, of the current insulin sensitivity of the patient, of a predicted blood glucose concentration as defined above, and of the glycemic target.

In particular, in basal infusion mode, the aggressiveness of the expert module 21 and the aggressiveness of the model predictive control module 20 are equal to one another, which allows to apply the same level of aggressiveness in basal infusion mode, regardless of the module used: $\alpha_e = \alpha_m = \alpha$.

Control of the bolus by the Expert module controller

According to one example, the expert module 21 operates in bolus infusion mode. "Bolus" is used to define a mode of infusion of insulin where the control parameter of the insulin delivery is a quantity of insulin, rather than a flow of insulin. Under this module, the aggressiveness is taken into account according to the following formula:

$$Bolus = \alpha_b\ .\ h,$$

where:

Bolus is the value of the control parameter,

$\alpha_b$ is the aggressiveness factor,

h is determined as the outcome of the expert module 21, which does not take into account the aggressiveness.

In particular, h may be determined by the expert module 21 based on one or more of the following parameters: a predicted blood glucose concentration value, a target or a target range, and a patient-specific value for the sensitivity to insulin. In particular, according to one embodiment, the patient-specific value for the sensitivity to insulin s is a multiplicative factor of an outcome $h_0$ of the expert module 21 which does not take into account the sensitivity to insulin of the patient, whereby h may be written $h = s \cdot h_0$. In this case, Bolus may be written as :

$$Bolus = \alpha_b \cdot s \cdot h_0,$$

whereby aggressiveness might be taken into account directly by modifying a patient-specific value for a factor $s_b$ of the sensitivity to insulin $s_b = a_b \cdot s$.

In particular, the aggressiveness factor in bolus infusion mode $\alpha_b$ might be different from the aggressiveness value in basal infusion mode or, in case of various aggressiveness values in basal infusion mode, from one or more or all of the various aggressiveness values in basal infusion mode. Because a patient might not be willing to experience a similar behavior in basal infusion mode and in bolus infusion mode, setting different values for the aggressiveness in the various modes may offer a better control to the patient. However, in a different variant, the aggressiveness factor in bolus infusion mode $\alpha_b$ might be equal to the aggressiveness value in one of the basal infusion modes. This would make the control simpler for the patient.

Control of the delivery by the activity-handling module controller

According to another example, the activity-handling module will now be described. In particular, the activity-handling module will be described in the context of a meal-handling module. However, the activity-handling module may handle other activities, such as physical exercise, sleep, ...

In the present example, the meal-handling module will be described as based on a declaratory module. Under a declaratory module, the patient is provided with an interface enabling him/her to provide the meal-handling module with information regarding a meal. Information may include various information enabling the meal-handling module to take into account the meal for the determination of insulin to be infused. Information may include one or more of:

- Occurrence of a meal (Y/N ?),

- Type of meal (Breakfast, lunch, dinner, snack, ...?),

- Time and/or duration of the meal (either past, present or predicted future),

- Nutritive information (estimated amount of CHO, ...).

The meal-handling module is not necessarily a declaratory module. It may be based on analysis of a blood glucose concentration curve.

The meal-handling module may determine a quantity of insulin to be infused to take into account the meal. This determination comes separate from the determination described above by the decision module 9, which is performed not taking into account any meal information.

In particular, the meal-handling module may operate in bolus mode. Under this module, the aggressiveness is taken into account according to the following formula:

$$MealBolus = \alpha_{mb} \cdot i,$$

where:

MealBolus is the value of the control parameter,

$\alpha_{mb}$ is the aggressiveness factor, which is a real parameter, and may depend on the type of meal (i.e. a declaration by the patient of a type of meal, as discussed above), and of the declared quantity of ingested carbohydrates, and/or the detected blood glucose concentration.

i is determined as the outcome of the meal-handling module, based at least on the target, which does not take into account the aggressiveness.

According to the present exemplary embodiment, $\alpha_{mb}$ might be either positive, null or negative. The present scheme ensures that a meal bolus will often be determined by the meal-handling module, except in exceptional circumstances where $\alpha_{mb} \cdot i$ will be negative and be larger than MB in absolute value. In particular, i will take into account the meal information.

According to this specific module, it may be provided that the amount of Meal Bolus is prompted to the patient using the user interface, and the user exerts a validation process in order to trigger injection of the insulin. The validation process may include the user amending the calculated MealBolus using the user interface.

Further, the meal-handling module may determine a schedule for distribution of the meal bolus. This determination may be based on the above input parameters. For example, the schedule may comprise a distribution of the meal bolus in two parts spaced in time. The volume of each part may be also determined by the meal-handling module. For example, the meal-handling module would determine a 60%-40% two-part distribution of the whole bolus.

The data processing system 3 regularly communicates with the active system 4 to send the determined value of the control parameter to the active system 4. The active system 4 receives the value of the control parameter, and controls its operation based on this value.

For example, if the determined value of the control parameter is to set an instantaneous flow debit of insulin to 1 U/h, it operates the pump at this debit.

This operation could be applied until a new command is received from the data processing system, or for a pre-defined period of time.

For example, if the determined value of the control parameter is to deliver an instantaneous quantity of insulin of 10 U, it operates the pump to deliver this quantity.

The above example is a three component system where an intermediate device comprises both the data processing system 3 and the user interface. However, other embodiments could be possible, such as, for example, having the data processing system 3 and the user interface in different devices, and/or incorporating one or the other in the data acquisition system 2 or in the active system 4.

The above description is based on data acquired by a data acquisition system 2. The data acquisition system may include one single blood glucose concentration sensor. Alternatively, it may include more than one blood glucose concentration sensors, located in various locations of the patient's body. Further, the data acquisition system may additionally comprise further data sensors, to acquire other relevant data of the patient. The data processing system 3 may also take into account not patient specific data. The data processing system 3 is adapted to determine the value for the control parameter based on the data acquired by the data acquisition system 2.

Even though the system above was described in relation to the infusion of insulin, other embodiments are foreseen, such as, for example, a controlling the infusion of insulin and of a counter-agent to insulin such as glucagon.

References

medical system 1
data acquisition system 2
data processing system 3
active system 4
hand-held device 5
decision module 9
model predictive control module 20
expert module 21
safety module 22
confidence assessment module 23
auto-learning module 25
activity-handling module 26
database 27
arrow 28, 29

**Claims**

1. A computerized system for repeatedly determining a value of a control parameter of a fluid infusion system for a diabetic patient, wherein the computerized system comprises a processor for repeatedly determining said value based on a data-based processing user-parametered with a target parameter and at least one aggressiveness parameter.

2. A computerized system according to claim 1, wherein the aggressiveness parameter relates to the acceleration of the action of the control parameter to force the value of the analyte toward the value of the target parameter.

3. Computerized system according to claim 1 or 2, wherein the processor determines a basal flow value by applying said data-based processing on detected analyte data.

4. Computerized system according to any of claims 1 to 3, wherein the processor determines a bolus quantity value.

5. Computerized system according to claim 4, wherein the processor determines a bolus value user-triggered to compensate for a meal.

6. Computerized system according to claim 5, wherein the processor determines a schedule for delivery of the bolus value.

7. Computerized system according to any of claims 1 to 6, wherein the processing comprises one or more of a model-predictive control module (20), an expert module (21), an activity-handling module (26), notably a meal-handling module.

8. Computerized system according to claim 7, wherein the processing comprises two or more of a model-predictive control module (20), an expert module (21), an activity-handling module (26), notably one or more meal-handling modules, wherein the aggressiveness parameter of two different modules are different, and notably wherein the aggressiveness parameter of two different modules determining a basal value are different and/or the aggressiveness factors of two different modules determining a bolus value are different.

9. A fluid infusion system for a diabetic patient comprising:

   - a sensor system (2) for acquiring patient data and storing said patient data in a database,
   - a computerized system (3) according to any of claims 1 to 8, and,
   - a pump (4) controlling infusion of said fluid into the patient based on said control parameter.

10. A fluid infusion system according to claim 9, further comprising a user interface adapted to enable parametring said target parameter and said at least one aggressiveness parameter.

11. A computer program adapted to repeatedly determine a value of a control parameter of a fluid infusion system for a diabetic patient, by making a processor repeatedly determining said value based on a data-based processing user-parametered with a target parameter and at least one aggressiveness parameter.

[Fig. 1]

[Fig. 2]

[Fig. 3]

Europäisches
Patentamt

European
Patent Office

Office européen
des brevets

## EUROPEAN SEARCH REPORT

Application Number

EP 20 20 1621

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/101225 A1 (O'CONNOR JASON [US] ET AL) 2 April 2020 (2020-04-02)<br>* figure 2 *<br>* paragraph [0015] - paragraph [0016] *<br>* paragraph [0023] *<br>* paragraph [0049] *<br>* paragraph [0087] *<br>* paragraph [0068] *<br>* paragraph [0071] - paragraph [0075] *<br>* paragraph [0083] *<br>* paragraph [0065] * | 1-11 | INV.<br>A61B5/00<br>G16H20/17<br>G16H40/63 |
| X,D | WO 2017/209902 A1 (ROCHE DIABETES CARE INC [US]; F HOFFMANN-LA ROCHE AG [CH] ET AL.) 7 December 2017 (2017-12-07)<br>* paragraph [0007] - paragraph [0008] *<br>* paragraph [0065] - paragraph [0071] * | 1-11 | |
| A | US 2019/247578 A1 (DESBOROUGH LANE [US] ET AL) 15 August 2019 (2019-08-15)<br>* paragraph [0007] - paragraph [0079] *<br>* paragraph [0118] - paragraph [0120] * | 1-11 | |
| A | US 2020/101223 A1 (LINTEREUR LOUIS J [US] ET AL) 2 April 2020 (2020-04-02)<br>* paragraph [0062] - paragraph [0072] * | 1-11 | TECHNICAL FIELDS SEARCHED (IPC)<br><br>A61B<br>G16H |

The present search report has been drawn up for all claims

| Place of search | Date of completion of the search | Examiner |
|---|---|---|
| Munich | 11 March 2021 | Hauber, Jörg |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.....................................................................................................
& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 20 20 1621

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2021

| Patent document cited in search report | | Publication date | Patent family member(s) | | Publication date |
|---|---|---|---|---|---|
| US 2020101225 | A1 | 02-04-2020 | US | 2020101225 A1 | 02-04-2020 |
| | | | WO | 2020069406 A1 | 02-04-2020 |
| WO 2017209902 | A1 | 07-12-2017 | AU | 2017275392 A1 | 03-01-2019 |
| | | | BR | 112018074903 A2 | 06-03-2019 |
| | | | CA | 3025906 A1 | 07-12-2017 |
| | | | CN | 109564774 A | 02-04-2019 |
| | | | EP | 3465491 A1 | 10-04-2019 |
| | | | KR | 20190013921 A | 11-02-2019 |
| | | | US | 2017348483 A1 | 07-12-2017 |
| | | | WO | 2017209902 A1 | 07-12-2017 |
| US 2019247578 | A1 | 15-08-2019 | AU | 2017207484 A1 | 28-06-2018 |
| | | | CA | 3009351 A1 | 20-07-2017 |
| | | | CN | 108495665 A | 04-09-2018 |
| | | | EP | 3374004 A1 | 19-09-2018 |
| | | | EP | 3443998 A1 | 20-02-2019 |
| | | | EP | 3453414 A1 | 13-03-2019 |
| | | | HK | 1257093 A1 | 11-10-2019 |
| | | | JP | 2019509074 A | 04-04-2019 |
| | | | US | 2017203037 A1 | 20-07-2017 |
| | | | US | 2017203038 A1 | 20-07-2017 |
| | | | US | 2017203039 A1 | 20-07-2017 |
| | | | US | 2017232195 A1 | 17-08-2017 |
| | | | US | 2019247578 A1 | 15-08-2019 |
| | | | US | 2020376197 A1 | 03-12-2020 |
| | | | US | 2021030955 A1 | 04-02-2021 |
| | | | US | 2021060245 A1 | 04-03-2021 |
| | | | WO | 2017124006 A1 | 20-07-2017 |
| US 2020101223 | A1 | 02-04-2020 | CN | 111246899 A | 05-06-2020 |
| | | | US | 2020101223 A1 | 02-04-2020 |
| | | | WO | 2020068189 A1 | 02-04-2020 |

EPO FORM P0459

For more details about this annex : see Official Journal of the European Patent Office, No. 12/82

**EP 3 984 443 A1**

**Patent documents cited in the description**

- WO 2017209902 A
- WO 2017205380 A